# EUROPÄISCHE PATENTSCHRIFT

(11) **EP 1 225 454 B1**
(45) Veröffentlichungstag und Bekanntmachung des Hinweises auf die Patenterteilung: **19.10.2011**
(21) Anmeldenummer: 02000006.3
(22) Anmeldetag: 02.01.2002
(51) Int. Cl.: G01S 5/16, A61B 19/00, A61B 17/00

(54) **Verfahren und Vorrichtung zum Festlegen eines Ortes**
Method and device for fixing a position
Procédé et dispositif de fixation d'une position

(30) Priorität: 03.01.2001 DE 10100335
(43) Veröffentlichungstag der Anmeldung: 24.07.2002
(73) Patentinhaber: Carl Zeiss Meditec AG, 07745 Jena (DE); Carl-Zeiss-Stiftung trading as Carl Zeiss, 89518 Heidenheim (Brenz) (DE)
(72) Erfinder: Hauger, Christoph, Dr., 73431 Aalen (DE); Pöltinger, Werner, 73447 Oberkochen (DE); Reimer, Peter, 73479 Ellwangen (DE); Krause-Bonte, Margit, 73432 Aalen (DE); Lasser, Theo, 1162 St. Prex (CH)
(74) Vertreter: Schorr, Frank Jürgen

(56) Entgegenhaltungen:
- WO-A-96/36897
- US-A- 5 657 128
- US-A- 5 662 111
- US-A- 5 694 142
- US-A- 5 704 897
- US-A- 5 795 294
- US-A- 5 795 295
- US-A- 5 841 149
- US-A- 5 871 445
- US-A- 5 889 611
- US-A- 6 081 370
- US-A- 6 146 390

## Beschreibung

Die Erfindung betrifft ein Verfahren und eine Vorrichtung zum Festlegen eines Ortes in einem Untersuchungsfeld, insbesondere in einem medizinischen Operationsfeld. Insbesondere betrifft die Erfindung auch die Gewinnung von Daten in Abhängigkeit von dem festgelegten Ort sowie deren Darstellung für einen Benutzer.

Aus US 5,795,295 ist ein Operationsmikroskop bekannt, welches eine Vorrichtung zur optischen Kohärenztomographie (OCT) umfasst, mit welcher ein Operateur, der ein Untersuchungsfeld durch das Mikroskop betrachtet, ergänzende Daten aus dem Operationsfeld gewinnen kann, wobei die Daten dann auf einem Monitor dargestellt werden. Ferner ist es bekannt, ergänzende Daten aus dem Untersuchungsfeld mittels einer Röntgen-Abbildungseinrichtung, einer Röntgen-Tomographieeinrichtung (CT) oder einer Kernspinresonanztomographieeinrichtung (NMR) zu erhalten.

US 5,795,294 offenbart ein Verfahren zum Korrelieren von verschiedenen Koordinatensystemen in computerunterstützter, stereotaktischer Operation. Dabei wird vor der Operation ein diagnostischer Datensatz aufgenommen und mit während einer Operation gewonnener topographischer Information korreliert.

Es ist eine Aufgabe der vorliegenden Erfindung, ein Verfahren und eine Vorrichtung anzugeben, um Möglichkeiten zur Gewinnung von Daten aus einem Untersuchungsfeld zu erweitern und zu präzisieren.

Insbesondere ist es eine Aufgabe der Erfindung, ein Verfahren und eine Vorrichtung anzugeben, um einen Abstand zwischen zwei Punkten eines Untersuchungsfelds genauer zu bestimmen.

Ferner ist es eine Aufgabe der Erfindung, die Gewinnung von Daten aus einem Untersuchungsfeld und/oder deren Darstellung für einen Benutzer vereinfacht zu gestalten.

Unter einem ersten Aspekt betrifft die Erfindung zunächst das Festlegen eines Ortes in einem Untersuchungsfeld bezüglich eines Koordinatensystems, wobei nachfolgend zu der Festlegung des Ortes anhand des festgelegten Ortes weitere Bestimmungen, Untersuchungen oder Verrichtungen vorgenommen werden. Hierbei kann ein Problem darin bestehen, dass sich das Untersuchungsfeld relativ zu dem Koordinatensystem bewegt oder sich ein Gesichtsfeld eines das Untersuchungsfeld betrachtenden Benutzers relativ zu dem Koordiantensystem bewegt, so dass nach einer solchen Bewegung Koordinaten des festgelegten Ortes nicht mehr ohne weiteres bestimmbar sind.

Die Erfindung sieht hierbei vor, zunächst Koordinaten eines festzulegenden Ortes in dem Untersuchungsfeld als Koordinatensatz zu gewinnen, der dem festzulegenden Ort zugeordnet ist. Hierzu muss ein Benutzer den festzulegenden Ort zunächst bezeichnen, indem er beispielsweise das Untersuchungsfeld durch ein Mikroskop betrachtet und dieses zum Untersuchungsfeld derart ausrichtet, dass der festzulegende Ort in einem Fadenkreuz oder Zentrum des Gesichtsfelds liegt, worauf er eine entsprechende Eingabe tätigt. Diese Eingabe ist eine Aufforderung dazu, die Koordinaten des Ortes des Untersuchungsfelds, der im Zentrum des Gesichtsfelds liegt, als Koordinatensatz zu bestimmen. Daraufhin wird eine erste Aufnahme topologischer Daten in einem räumlich ausgedehnten Bereich nahe dem identifizierten Ort des Untersuchungsfelds gewonnen. Nach einer gewissen Zeitdauer wird eine zweite Aufnahme topologischer Daten in diesem Bereich gewonnen. Sollte sich während dieser Zeit das Untersuchungsfeld bezüglich des Koordinatensystems verlagert haben, so ist es durch einen Vergleich der beiden Aufnahmen möglich, die Größe der Verlagerung des identifizierten Ortes bezüglich des Koordinatensystems zu ermitteln. Daraufhin werden die Koordinaten des Koordinatensatzes in Abhängigkeit von der ermittelten Verlagerung derart abgeändert, dass die Koordinaten des Koordinatensatzes mit den Koordinaten des identifizierten Orts im Untersuchungsfeld nach dessen Verlagerung im Wesentlichen übereinstimmen. Es ist somit möglich, einen identifizierten Ort im Untersuchungsfeld zu verfolgen, so dass nach einer Verlagerung des identifizierten Orts dieser nicht erneut identifiziert werden muss.

Hierbei ist es vorteilhaft, zeitlich wiederkehrend weitere Aufnahmen topologischer Daten zu gewinnen und durch Vergleich der gewonnen Aufnahmen miteinander eine aktuelle Verlagerung des identifizierten Orts bezüglich des Koordinatensystems zu ermitteln und die Koordinaten des Koordinatensatzes derart abzuändern, dass diese den aktuellen Koordinaten des identifizierten Orts im Untersuchungsfeld angeben.

Zur Identifizierung des festzulegenden Ortes kann der Benutzer direkt, d.h. mit "unbewaffnetem" Auge auf das Untersuchungsfeld blicken, oder er kann sich zur Betrachtung des Untersuchungsfelds eines abbildenden Geräts bedienen, welches eine Kamera und einen Bildschirm oder ein Mikroskop oder eine Brille, insbesondere eine Lupenbrille, umfasst.

Zur Identifizierung des Ortes ist vorteilhafterweise ein Zeiger mit einer Zeigerspitze vorgesehen, die der Benutzer mit dem identifizierten Ort in Kontakt bringen kann. Dann ist ferner eine Positionserfassungsvorrichtung zur Bestimmung der Position der Zeigerspitze bezüglich des Koordinatensystems vorgesehen.

Bedient sich der Benutzer eines abbildenden Geräts, so stellt dieses vorteilhafterweise ferner eine Markierung dar, die der Benutzer in seinem Gesichtsfeld in Deckung mit dem zu identifizierenden Ort bringen kann, um diesen zu bezeichnen. Diese Markierung kann beispielsweise eine fest im Gesichtsfeld des abbildenden Geräts angeordnete Markierung, wie etwa ein Fadenkreuz, sein, oder die Markierung ist durch den Benutzer im Gesichtsfeld des abbildenden Geräts verschiebbar.

Vorteilhafterweise ist ebenfalls eine Blickrichtungserfassungseinrichtung vorgesehen, welche aus einer Augenstellung des Benutzers dessen Blickrichtung erfasst, so dass der Benutzer den festzulegenden Ort einfach durch seine Augenstellung, d.h. seine Blickrichtung, identifizieren kann.

Bei all den geschilderten und auch weiteren Ausgestaltungen der Identifizierung des festzulegenden Orts werden die Koordinaten des identifizierten Ortes auf eine entsprechende Aufforderung durch den Benutzer hin in den Koordinatensatz übernommen. Diese Aufforderung durch den Benutzer kann durch Betätigen eines elektrischen oder mechanischen Schalters, durch eine Sprachaufforderung oder durch eine Augenbewegung des Benutzers erfolgen.

Nach der Identifizierung des festzulegenden Ortes kann sich der Benutzer weiteren Tätigkeiten zuwenden, wobei der identifizierte Ort auch bei Verlagerungen verfolgt wird, indem der Koordinatensatz derart abgeändert wird, dass dessen Koordinaten mit den Koordinaten des identifizierten Orts im Wesentlichen übereinstimmen. Hierbei ist es ferner möglich, den Koordinatensatz einer Datenerfassungsvorrichtung zuzuführen, welche an dem durch den Koordinatensatz angegebenen Ort, d.h. im Wesentlichen an dem identifizierten Ort, Messungen vornimmt, um wenigstens eine im Zusammenhang mit dem Untersuchungsfeld stehende Größe zu bestimmen, welche vorteilhafterweise in das Blickfeld des Benutzers eingeblendet wird.

Ferner ist es möglich, nach dem vorangehend erläuterten identifizierten Ort einen oder mehrere weitere Orte des Untersuchungsfelds zu identifizieren und gegebenenfalls auch diese weiteren Orte über Aufnahmen topologischer Daten auch bei Verlagerungen zu verfolgen. Eine vorteilhafte Anwendung ist dadurch gegeben, dass die bestimmte Größe einen Wert umfasst, der im Wesentlichen einen Abstand zweier identifizierter Orte voneinander angibt.

Ferner ist es vorteilhaft, dass die wenigstens eine bestimmte Größe einen Datensatz umfasst, der aus einer Mehrzahl von Messungen gewonnen ist, die entlang einer Verbindungslinie zwischen zwei identifizierten Orten durchgeführt werden. Der Datensatz kann beispielsweise ein entlang der Verbindungslinie aufgenommenes Tiefenprofil des Untersuchungsfelds oder einen mittels eines räumlichen, bildgebenden Verfahrens, wie beispielsweise Computertomografie, gewonnenen Schnitt umfassen, dessen Darstellung vorteilhafterweise ebenfalls in das Gesichtsfeld des Benutzers eingeblendet wird.

Die topologischen Daten umfassen vorteilhafterweise Koordinaten einer Mehrzahl von Oberflächenpunkten des Untersuchungsfeldes, die beispielsweise mittels Lasertriangulation oder Ähnlichem gewonnen werden können. Es ist ebenfalls möglich, die Aufnahmen topologischer Daten mittels einer Kamera, wie etwa einer CCD-Kamera, zu gewinnen und diese Aufnahmen zur Bestimmung der Verlagerung des Orts im Untersuchungsfeld zu vergleichen.

Ferner ist es ebenfalls vorteilhaft, als topologische Daten auch Messwerte zu verwenden, welche von Punkten unterhalb der Oberfläche des Untersuchungsfeldes gewonnen werden. Derartige Daten können beispielsweise mittels optischer Kohärenztomographie (OCT) oder Röntgen-Computertomografie (CT) oder Kernspintomographie (NMR) oder Ähnlichem gewonnen werden.

Unter einem weiteren Aspekt sieht die Erfindung eine Vorrichtung zum Festlegen wenigstens eines Ortes in einem Untersuchungsfeld vor, welche eine Vorrichtung zur Anzeige einer aus einem Untersuchungsfeld gewonnen Größe vorsieht, wobei die anzuzeigende Größe an einem vorab von dem Benutzer identifizierten Ort des Untersuchungsfeldes, in der Umgebung eines solchen Ortes oder in Abhängigkeit von einem solchen Ort bestimmt wird. Hierbei ist vorgesehen, dass der Benutzer den zu identifizierenden Ort mit seinen Augen bestimmen kann, indem eine Blickrichtungserfassungsvorrichtung zur Bestimmung der Augenstellung des Benutzers vorgesehen ist. Die im Zusammenhang mit dem identifizierten Ort zu bestimmende Größe wird durch die Vorrichtung sodann erfasst und in das Blickfeld des Benutzers derart eingeblendet, dass sowohl die Darstellung der gewonnenen Messgröße als auch das Untersuchungsfeld selbst gleichzeitig im Blickfeld des Benutzers liegen. Hierdurch ist zum Einen eine besonders einfache Identifizierung eines Ortes im Untersuchungsfeld durch den Benutzer möglich, und zum Anderen kann der Benutzer die in Abhängigkeit von dem identifizierten Ort gemessene Größe in seinem Gesichtsfeld wahrnehmen, ohne seinen Blick von dem Untersuchungsfeld wegwenden zu müssen, wie etwa hin zu einer herkömmlichen Darstellung der Messgröße auf einem außerhalb des Untersuchungsfelds angeordneten Bildschirm.

Hierzu wird die Darstellung der Größe vorteilhafterweise in einen Strahlengang eines Mikroskops oder einer Lupenbrille eingeblendet, mit der der Benutzer das Untersuchungsfeld betrachtet.

Vorteilhafterweise ist hierbei eine Positionserfassungseinrichtung vorgesehen, um die Position der Lupenbrille bzw. des Mikroskops bezüglich des Koordinatensystems zu erfassen, so dass über die Blickrichtungserfassungseinrichtung auch die Koordinaten des Ortes ermittelt werden können, auf welchen der Blick des Benutzers weist.

Ausführungsformen der Erfindung werden nachfolgend anhand von Zeichnungen erläutert. Hierbei zeigt:
- Figur 1: ein Operationssystems, bei dem ein Benutzer durch ein Mikroskops auf ein Untersuchungsfeld blickt,
- Figur 2: eine schematische Darstellung eines Gesichtsfelds gesehen durch das Mikroskop der Figur 1,
- Figur 3: ein mittels einer Kohärenztomographievorrichtung der Figur 1 gewonnenes Tiefenprofil an einem Punkt des Untersuchungsfelds und
- Figur 4: eine Darstellung eines Flussdiagramms zur Arbeitsweise der Vorrichtung gemäß Figur 1.

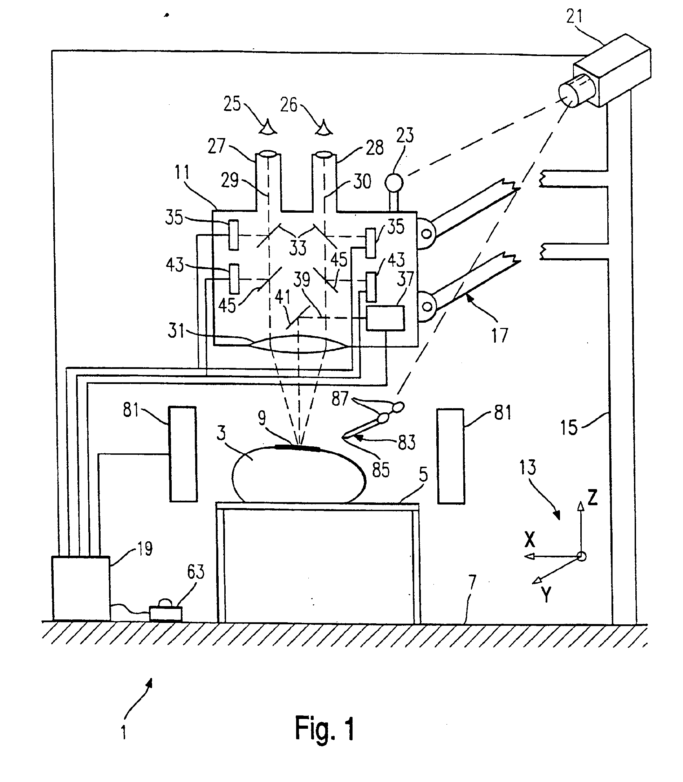

In Figur 1 ist ein Operationssystem 1 zur Durchführung mikrochirurgischer Operationen an einem Patienten schematisch dargestellt. Ein zu operierender Körperteil 3 des Patienten ist auf einem Operationstisch 5 gelagert, der fest auf einem Boden 7 eines Operationssaales steht. Ein Untersuchungs- bzw. Operationsfeld 9 des Körperteils 3 wird von dem Operateur durch ein Stereo-Operationsmikroskop 11 betrachtet, welches in einem Koordinaten-Bezugssystem 13 des Operationssaales an einem Stativ 15 mittels einer Schwenkhalterung 17 in den drei Raumrichtungen x, y, z verlagerbar ist. Eine aktuelle Position des Mikroskops 11 in dem Koordinatensystem 13 wird mittels einer Positionserfassungseinrichtung bestimmt und an einen Rechner 19 übermittelt. Die Positionserfassungseinrichtung umfasst eine fest an dem Mikroskop 11 angebrachte Lichtquelle 23, wie etwa eine Leuchtdiode, deren Position in dem Koordinatensystem 13 von drei mit Abstand voneinander angeordneten Kameras erfasst wird, von denen in Figur 1 lediglich eine Kamera 21 dargestellt ist, die wiederum fest an dem Stativ 15 montiert ist.

Der Operateur blickt mit seinen Augen 25, 26 in Okulartuben 27, 28 des Operationsmikroskops 11, wobei den beiden Augen 25, 26 zugeordnete Stereo-Strahlengänge 29, 30 durch die beiden Okulartuben 27, 28 und eine gemeinsame Objektivlinse 31 auf das Operationsfeld 9 verlaufen und dort fokussiert sind.

In einem jeden der Stereo-Strahlengänge 29, 30 ist ein halbdurchlässiger Spiegel 33 angeordnet, der ein Bild der Pupille des Auges aus dem Strahlengang 29, 30 auskoppelt und auf jeweils eine CCD-Kamera 35 wirft. Die von den Kameras 35 registrierten Bilder werden dem Rechner 19 zugeführt, dort analysiert und daraus eine Blickrichtung der beiden Augen 25, 26 des Operateurs ermittelt.

Ein ähnliches Mikroskop mit Blickrichtungserfassung ist in EP 0 788 613 B1 beschrieben, deren Offenbarung in die vorliegende Anmeldung vollumfänglich einbezogen wird.

Das Operationsmikroskop 11 umfasst ferner eine Vorrichtung 37 zur Durchführung optischer Kohärenztomographie (OCT), deren Messstrahl 39 über einen Spiegel 41 in den Strahlengang des Mikroskops 11 eingekoppelt und durch das Objektiv 31 in dem Operationsfeld 9 fokussiert wird. Ein Mikroskop mit OCT-Vorrichtung ist beispielsweise aus US 5 795 295 bekannt, deren Offenbarung durch Inbezugnahme ebenfalls in die vorliegende Anmeldung vollumfänglich aufgenommen wird.

Durch die OCT-Vorrichtung 37 kann der Messstrahl scannend auf jeden Punkt im Blickfeld des Mikroskops 11 gerichtet werden, um von diesem Punkt OCT-Messdaten zu gewinnen.

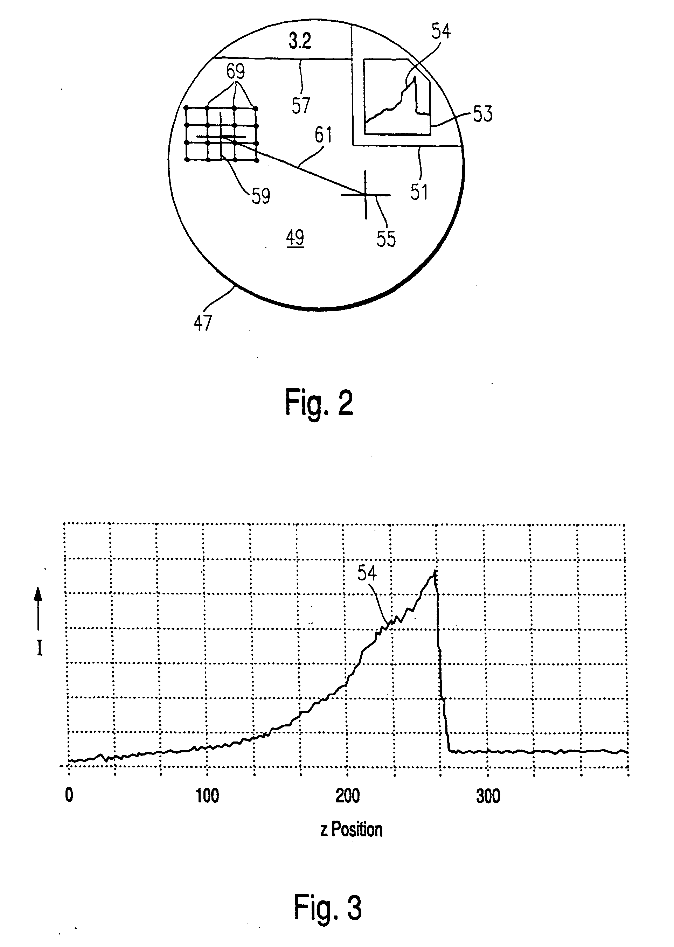

Ein Beispiel für solche Messdaten 54 ist in Figur 3 dargestellt, in der eine reflektierte Intensität I des Messstrahls 39 in Abhängigkeit von einer Entfernung z von dem Mikroskop 11 aufgetragen ist. Die steile Flanke bei z = 265 repräsentiert hierbei die Oberfläche des Körperteils 3 im Untersuchungsfeld 9, und die zu niedrigeren z-Werten hin abfallende Flanke repräsentiert die in Abhängigkeit von der Tiefe des Körperteils 3 abnehmende reflektierte Intensität des Messstrahls 39. Die Eindringtiefe des Messstrahls 39 in das Gewebe des Körperteils 3 beträgt etwa 2 bis 3 mm.

Die OCT-Vorrichtung 37 wird von dem Rechner 19 derart angesteuert, dass der Ort in dem Untersuchungsfeld 9, an dem die OCT-Vorrichtung 37 die Messdaten gewinnt, mit dem Ort zusammenfällt, auf den der Blick des Operateurs gerichtet ist. Die an diesem Ort gewonnenen Messdaten werden von der OCT-Vorrichtung 37 wiederum an den Rechner 19 übermittelt.

Das Mikroskop 11 umfasst ferner zwei LCD-Anzeigen 43, deren Bilder jeweils über halbdurchlässige Spiegel 45 in die Stereo-Strahlengänge 29, 30 derart eingekoppelt werden, dass sie im Blickfeld des Operateurs erscheinen. Ein Beispiel für die Einkopplung von Bildern in den Strahlengang eines Mikroskops ist ebenfalls in der hier einbezogenen EP 0 788 613 B1 erläutert.

Ein Beispiel für eine Darstellung für ein Bildfeld 47 des Mikroskops 11, wie es der Operateur wahrnimmt, ist in Figur 2 dargestellt. Hierbei ist ein Teilfeld 49 des Bildfelds 47 für die Darstellung des Operationsfelds 9 unbeeinflusst durch die Anzeigen 43 vorgesehen. In einem rechts oben in dem Bildfeld 47 angeordneten Teilfeld 51 erzeugen die Anzeigen 43 eine Darstellung 53 der von der OCT-Vorrichtung 37 gewonnenen OCT-Messdaten 54. Diese OCT-Messdaten 54 werden, wie vorangehend beschrieben, an dem Ort des Operationsfelds 9 gewonnen, auf den der Blick des Operateurs gerichtet ist, wobei dieser Ort zur Verdeutlichung in dem Teilfeld 49 durch ein Markierungskreuz 55 gekennzeichnet ist, welches ebenfalls rechnergesteuert durch die Anzeigen 43 für den Operateur sichtbar gemacht wird.

Durch das Operationssystem 1 ist somit eine sehr einfache Möglichkeit gegeben, OCT-Messdaten 54 aus einem Operationsfeld zu gewinnen, wobei der Ort zur Aufnahme der OCT-Messdaten 54 durch die Blickrichtung des Operateurs vorgegeben ist und die gewonnenen Messdaten zeitgleich in das Gesichtsfeld 47 des Operateurs eingeblendet werden.

In einem Teilbereich 57 des Bildfelds 47 des Mikroskops 11 blenden die Anzeigen 43 ferner eine numerische Darstellung (3,2) eines Abstands D in Millimetern zwischen zwei verschiedenen durch den Operateur identifizierten Orten des Untersuchungsfelds 9 ein. Die beiden Orte, deren Abstand D voneinander in dem Teilfeld 57 eingeblendet wird, sind zum Einen der durch das Markierungskreuz 55 markierte Ort, auf den der Blick des Operateurs gerade gerichtet ist, sowie zum Anderen ein vorab von dem Operateur bestimmter Ort, dessen Lage im Gesichtsfeld 47 durch ein weiteres Markierungskreuz 59 dargestellt ist und durch die Anzeigen 43 in dieses eingeblendet wird. Die Anzeigen 43 blenden weiter eine gerade Verbindungslinie 61 zwischen den beiden Orten 55 und 59 in das Gesichtsfeld 47 ein.

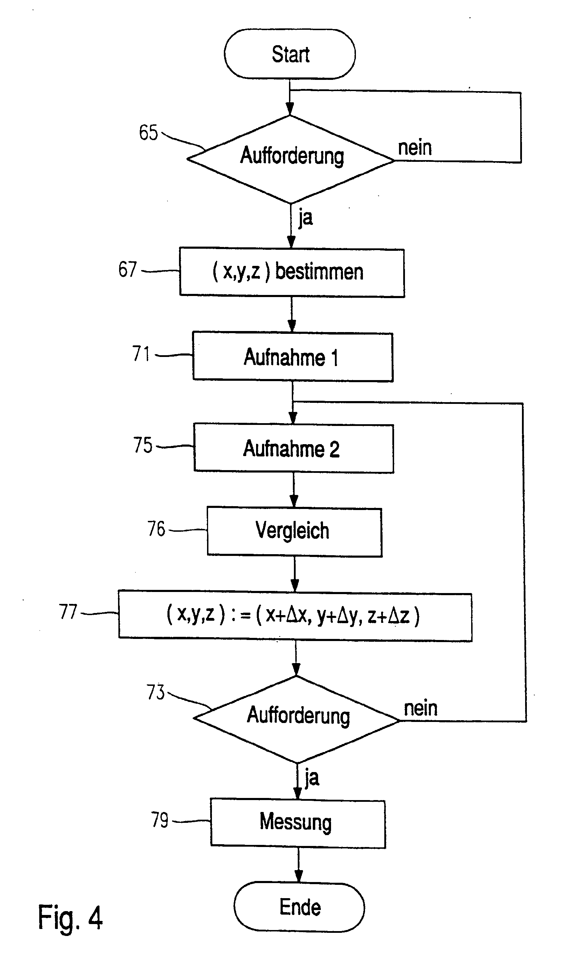

Die Identifizierung des durch das Kreuz 59 markierten Ortes durch den Operateur sowie die Darstellung der Lage des Kreuzes 59 im Bildfeld 47 des Mikroskops erfolgte nach dem im Zusammenhang mit Figur 4 nachfolgend erläuterten Verfahren.

Der Operateur identifiziert diesen Ort durch seine Blickrichtung und Betätigung eines Fussschalters 63 (Figur 1). Das System wartet hierbei in einem Schritt 65 auf eine Aufforderung, die der Operateur durch die Betätigungs des Fussschalters gibt. Auf die Aufforderung hin bestimmt das System in einem Schritt 67 einen Koordinatensatz (x, y, z) der Koordinaten des Ortes in dem Bezugssystem 13, auf den der Blick des Operateurs gerichtet ist. In diese Bestimmung geht die durch die Positionserfassungseinrichtung 21, 23 erfasste Lage des Mikroskops 11 in dem Koordinatensystem 13 sowie weiter die durch die Blickrichtungerfassungseinrichtung 35 ermittelte Blickrichtung des Operateurs und ein Abstand des Ortes, auf den der Blick des Operateurs gerichtet ist, von dem Mikroskop 11 ein, welcher Abstand mittels der OCT-Vorrichtung 37 erfasst wird.

Nach der Bestimmung des Koordinatensatzes (x, y, z) für den von dem Operateur identifizierten Ort stellt der Rechner 19 diesen Ort in dem Gesichtsfeld 47 durch das Kreuz 59 dar und nimmt in einem Schritt 71 zugleich eine Aufnahme topologischer Daten eines um den identifizierten Ort räumlich ausgedehnten Bereichs auf. Die Aufnahme topologischer Daten umfasst OCT-Aufnahmen von 16 Messpunkten 69, die nach Art eines Gitters um den identifizierten Ort herum angeordnet sind, wie dies in Figur 2 angedeutet ist, wobei die Punkte 69 allerdings nicht in das Gesichtsfeld 47 eingeblendet werden. Hierdurch gewinnt das Operationssystem Kenntnis über die Topologie des von dem Operateur identifizierten Ortes und ist in der Lage, diesen Ort zu verfolgen, selbst wenn sich das Untersuchungsfeld 9 beispielsweise durch Atembewegung des Patienten verlagert, oder wenn der Operateur das Mirkoskop 11 relativ zum Patienten verlagert. Eine solche Verlagerung kann hierbei sowohl eine translatorische Verlagerung als auch eine rotatorische Verlagerung sein.

Hierzu durchläuft das System gemäß dem Flussdiagramm der Figur 4 eine Schleife, welche in einem Schritt 73 dann verlassen wird, wenn der Operateur hierzu eine weitere Aufforderung über den Fussschalter 63 gibt. Innerhalb dieser Schleife wird in einem Schritt 75 zunächst eine zweite Aufnahme topologischer Daten an den gleichen Koordinatenpunkten 69 gewonnen, wie dies für die erste Aufnahme in dem Schritt 71 der Fall war. In einem Schritt 76 führt das System einen Vergleich der beiden Aufnahmen topologischer Daten durch und ermittelt hieraus eine Verlagerung (Δx, Δy, Δz) des Untersuchungsfelds 9 bezüglich des Koordinatensystems 13, welche gegebenenfalls in dem Zeitraum zwischen den beiden Aufnahmen (zwischen den Schritten 71 und 75) stattgefunden hat. Aus dieser Verlagerung (Δx, Δy, Δz) können damit die Koordinaten des von dem Operateur identifizierten Orts nach der Verlagerung bestimmt werden, und in einem Schritt 77 werden die Koordinaten des Koordinatensatzes (x, y, z) entsprechend abgeändert, wobei auch die Lage des Markierungskreuzes 59 in dem Gesichtsfeld 47 entsprechend aktualisiert wird.

Somit kann der Operateur für die gewünschte Abstandsbestimmung zweier Punkte zunächst den ersten der beiden Punkte identifizieren und sich dann der Identifizierung des zweiten Punktes ungestört zuwenden, da das System die Lage des ersten identifizierten Ortes auch bei Verlagerungen des Untersuchungsfelds selbsttätig verfolgt. Während der Identifizierung des zweiten Ortes, der in dem Gesichtsfeld 47 durch das Kreuz 55 markiert ist, wird der Abstand zu dem ersten Ort in dem Anzeigeteil 57 des Gesichtsfelds 47 ständig aktualisiert, wobei OCT-Messdaten von dem zweiten Ort in dem Anzeigeteil 51 ebenfalls ständig eingeblendet werden.

Ist der zweite Ort zur Zufriedenstellung des Operateurs festgelegt, so betätigt er den Fussschalter 63 erneut, worauf im Schritt 73 die vorangehend beschriebene Schleife verlassen wird und in einem Schritt 79 abschließend eine Messung in Abhängigkeit von den beiden identifizierten Orten (59 und 55) durchgeführt wird. Diese Messung kann einen OCT-Scan entlang der Linie 61 zwischen den beiden Orten 59 und 55 umfassen, wobei eine bildliche Darstellung der entsprechenden Messdaten in den Anzeigeteil 51 des Gesichtsfelds 47 eingeblendet wird.

Ergänzend kann auch der zweite Ort, gleichzeitig mit dem ersten Ort nach der vorangehend beschriebenen Methode, also durch Aufnahme mehrerer topologischer Daten aus der Umgebung des zweiten Orts und durch Vergleich derselben miteinander, verfolgt werden, sodass der Operateur beispielsweise die Blickrichtung des Mikroskops ändern kann oder sonstige Verrichtungen vornehmen kann, bevor die Messung in Abhängigkeit der beiden Orte durchgeführt wird. Ferner können nach dem zweiten Ort noch weitere Orte identifiziert werden und Messungen in Abhängigkeit der identifizierten drei oder mehr Orte durchgeführt werden.

Alternativ oder ergänzend zu dem OCT-Scan entlang der Linie 61 kann die in dem Schritt 79 durchgeführte Messung in Abhängigkeit von den beiden bestimmten Punkten ebenfalls jegliche andere Gewinnung von Messdaten umfassen, wie beispielsweise die Durchführung einer Röntgen-computertomographischen Aufnahme (CT) mittels einer in Figur 1 dargestellten Tomographievorrichtung 81, wobei der Rechner dann eine Schnittdarstellung der Tomographiedaten entlang der Verbindungslinie 61 liefert. Die Tomographievorrichtung 81 kann auch eine Kernspinresonanztomographie-Einrichtung sein, um eine entsprechende Kernspinresonanztomographieaufnahme (NMR) zu liefern.

Die vorangehend beschriebenen Messmethoden ergeben eine Darstellung eines Schnitts entlang der durch die beiden Punkte festgelegten Verbindungslinie 61, wobei die Orientierung des Schnittes in eine Richtung senkrecht zur Verbindungslinie 61 noch wählbar ist. Vorteilhafterweise fällt diese Richtung jedoch mit der Blickrichtung des Mikroskops zusammen. Wird auch, wie vorangehend beschrieben, der zweite identifizierte Punkt und damit beide die Verbindungslinie definierenden Punkte in Bezug auf Verlagerungen in dem Koordinatensystem oder bezüglich des Blickfelds des Betrachters verfolgt, so ist es möglich, nach Festlegen der Verbindungslinie 61 das Mikroskop zu verschwenken, um damit die Orientierung des Schnittes und dessen Darstellung im Blickfeld des Benutzers zu verändern.

Eine vorteilhafte Anwendung der vorangehend beschriebenen Abstandsbestimmung mittels der OCT-Vorrichtung 37 ist im Bereich von Mittelohroperation gegeben, um Abmessungen von Implantaten, insbesondere einer Stapesprothese, zu vermessen.

Alternativ zur Gewinnung der Aufnahme topologischer Daten an den in einem Gitter angeordneten Messpunkten 69 können diese Messpunkte auch in einer beliebigen anderen Konfiguration um den identifizierten Ort 59 gewonnen werden, wie beispielsweise entlang einer Kreislinie um den identifizierten Ort angeordneten Messpunkten.

Die Aufnahme topologischer Daten kann auch nach anderen Methoden als mit der OCT-Vorrichtung 37 erfolgen. Beispielsweise können die topologischen Daten auch mittels der Tomografievorrichtung 81 gewonnen werden, oder es kann die Aufnahme topologischer Daten auch durch eine Kamera gewonnen werden, die auf das Untersuchungsfeld 9 gerichtet ist. Diese Kamera kann auch in den Strahlengang des Mikroskops 11 integriert sein, beispielsweise indem diese, wie die OCT-Vorrichtung 37, über den Spiegel 41 in den Strahlengang des Mikroskops eingekoppelt ist.

Ferner ist es möglich, zwei mit Abstand voneinander angeordnete Kameras für die Gewinnung der topologischen Daten einzusetzen, deren Bilder photogrammetrisch, d. h. unter Ausnutzung der unter unterschiedlichen Blickwinkeln aufgenommenen Bilder der beiden Kameras ausgewertet werden.

Bei einer Gewinnung der topologischen Daten mittels einer Kamera werden vornehmlich Oberflächendaten des Untersuchungsfelds gewonnen, bei Verwendung der tomographischen Aufnahmegeräte, wie OCT-Vorrichtung 37 oder Tomographievorrichtung 81, werden vorzugsweise auch die durch diese Geräte bereitgestellten Volumendaten als topologische Daten verwendet.

Die Aufforderung zur Identifizierung der Orte im Untersuchungsfeld durch den Operateur wird, wie vorangehend beschrieben, durch Betätigung des Fussschalters 63 gegeben. Alternativ zu dem Fussschalter 63 ist jegliche andere Eingabeeinrichtung möglich. Insbesondere ist hierzu eine Spracheingabevorrichtung mit einem Mikrofon und einem entsprechenden Auswerteprogramm der Mikrofonsignale in dem Rechner 19 vorgesehen, so dass der Operateur die Aufforderung auch durch ein gesprochenes Wort geben kann. Ferner ist vorgesehen, dass die Blickrichtungserfassungsvorrichtung über die Kameras 35 beispielsweise ein willentlich herbeigeführtes Blinzeln der Augen des Operateurs erfasst, welches der Rechner 19 dann als Aufforderung zur Identifizierung des Ortes wertet. Somit kann die Aufforderung auch blickgesteuert erfolgen.

In den vorangehenden Ausführungsbeispielen wurde die Identifizierung des festzulegenden Ortes über die Blickrichtung des Operateurs beschrieben. Alternativ oder ergänzend hierzu ist auch eine Identifizierung des Ortes mittels einer Zeigervorrichtung 83 vorgesehen, die ähnlich einem Stift von dem Operateur in der Hand gehalten wird und eine Spitze 85 und an einem der Spitze 85 gegenüberliegenden Ende zwei Lichtquellen 87 umfasst, deren Lage im Koordinatensystem 13 durch die Kameras 21 erfasst wird. Der Operateur kann die Zeigevorrichtung 83 per Hand führen und die Spitze 85 in die Nähe des zu identifizierenden Ortes bringen. Nach Aufforderung durch beispielsweise den Fussschalter 63 ermittelt der Rechner 19 dann die Koordinaten des Ortes, auf den die Spitze 85 der Zeigevorrichtung 83 zeigt.

Alternativ zu dem Mikroskop 11, das an dem Stativ 15 bezüglich des Koordinatensystems 13 verlagerbar gehalten ist, kann der Operateur auch eine mittels einer Halterung am Kopf des Operateurs befestigte Lupenbrille tragen, welche, ähnlich wie das Mikroskop 11, die Blickrichtung des Operateurs erfasst und diesem über Bildschirme Darstellungen in sein Gesichtsfeld einblendet, wobei Lage und Orientierung der Lupenbrille im Koordinatensystem 13 ebenfalls durch eine Positionserfassungseinrichtung erfasst wird. Im Hinblick auf eine Gewichtseinsparung kann die Vorrichtung zur Aufnahme topologischer Daten dann separat von der Lupenbrille an einem Stativ im Operationssaal gehaltert sein.

Ferner ist es auch denkbar, dass der Operateur im Wesentlichen direkt auf das Untersuchungsfeld blickt, d. h. er blickt auf das Untersuchungsfeld nicht durch ein vergrößerndes abbildendes Gerät, dessen Bild er betrachtet. Der Operateur trägt dann eine Halterung für eine Dateneinspiegelung in sein Blickfeld, sodass er gleichzeitig die eingespiegelten Daten und das Untersuchungsfeld direkt in seinem Blickfeld hat. Die Identifizierung eines Ortes kann dann dadurch erfolgen, dass sich in dem Blickfeld des Operateurs eine Markierung, wie etwa ein Fadenkreuz, befindet und der Operateur den Kopf derart schwenkt, dass die Markierung auf den zu identifizierenden Ort des Untersuchungsfelds zeigt und er sodann die entsprechende Aufforderung gibt. Es ist hierdurch eine besonders einfache Möglichkeit für die Identifizierung von Orten bereitgestellt, da eine separate Blickrichtungserfassungseinrichtung nicht notwendig ist. Die Markierung kann entweder fest mit der Halterung verbunden sein, oder sie kann durch die Dateneinblendung bereitgestellt werden.

Im Übrigen kann auch bei dem vorangehend beschriebenen Mikroskop oder jedem anderen abbildenden Gerät, durch das der Operateur auf das Untersuchungsfeld blickt, die Markierung fest mit dem abbildenden Gerät verbunden sein, sodass der Operateur durch Bewegung des abbildenden Geräts die Markierung zur Deckung mit dem zu identifizierenden Ort bringen und sodann die entsprechende Aufforderung geben kann.

## Patentansprüche

1. Verfahren zum Festlegen wenigstens eines Ortes in einem Untersuchungsfeld (9) bezüglich eines Koordinatensystems (13), wobei nach einer Aufforderung (65) Koordinaten eines ersten Ortes (59) als ein dem ersten Ort (59) zugeordneter erster Koordinatensatz (x, y, z) bestimmt werden, und daraufhin
aufeinanderfolgend eine erste und eine zweite Aufnahme topologischer Daten (69) in einem räumlich ausgedehnten Bereich um den ersten Ort (59) im Untersuchungsfeld (9) gewonnen werden,
aus einem Vergleich der beiden Aufnahmen miteinander eine Verlagerung (Δx, Δy, Δz) des ersten Ortes (59) des Untersuchungsfelds (9) bezüglich des Koordinatensystems (13) bestimmt wird, und
die Koordinaten des dem ersten Ort zugeordneten ersten Koordinatensatzes (x, y, z) in Abhängigkeit von der bestimmten Verlagerung (Δx, Δy, Δz) derart abgeändert werden, daß sie mit den Koordinaten des ersten Orts (59) im Untersuchungsfeld (9) nach dessen Verlagerung im Wesentlichen übereinstimmen.

2. Verfahren nach Anspruch 1, wobei die Aufnahmen topologischer Daten (69) zeitlich wiederkehrend gewonnen und miteinander verglichen und daraus Verlagerungen (Δx, Δ y, Δz) bestimmt werden, um jeweils aktuelle Koordinaten des ersten Ortes (59) im Untersuchungsfeld (9) zu erhalten.

3. Verfahren nach Anspruch 1 oder 2, wobei in Abhängigkeit von dem ersten Koordinatensatz (x, y, z) wenigstens eine Größe bestimmt wird.

4. Verfahren nach Anspruch 3, wobei eine Darstellung (53) der wenigstens einen Größe (54) in ein Blickfeld (47) eines Benutzers eingeblendet wird.

5. Verfahren nach einem der Ansprüche 1 bis 4, wobei nach einer weiteren Aufforderung (73) Koordinaten eines zweiten Ortes (55) als ein dem zweiten Ort (55) zugeordneter zweiter Koordinatensatz bestimmt werden und in Abhängigkeit von den beiden Koordinatensätzen wenigstens eine Größe (D) bestimmt wird.

6. Verfahren nach Anspruch 5, wobei die wenigstens eine Größe einen Wert umfaßt, der im Wesentlichen einen Abstand (D) der beiden Orte (59, 55) voneinander angibt.

7. Verfahren nach Anspruch 5, wobei die wenigstens eine Größe einen Datensatz umfaßt, der aus einer Mehrzahl von Messungen gewonnen ist, die entlang einer Verbindungslinie (61) zwischen den beiden Orten (59, 55) an dem Untersuchungsfeld (9) durchgeführt werden.

8. Verfahren nach einem der Ansprüche 1 bis 7, wobei die Aufnahme topologischer Daten Koordinaten einer Mehrzahl von Oberflächenpunkten (69) des Untersuchungsfeldes (9) umfaßt.

9. Verfahren nach einem der Ansprüche 1 bis 8, wobei die Aufnahme topologischer Daten Meßwerte von Punkten (69) unterhalb der Oberfläche des Untersuchungsfeldes (9) umfaßt.

10. Verfahren nach einem der Ansprüche 1 bis 9, wobei mittels eines abbildenden Geräts (11) ein Bild (49) des Untersuchungsfeldes (9) zur Betrachtung durch einen Benutzer dargestellt wird oder der Benutzer direkt auf das Untersuchungsfeld (9) blickt.

11. Verfahren nach Anspruch 10, wobei zur Betrachtung durch den Benutzer ferner eine Markierung (55) dargestellt wird, deren Lage bezüglich des durch den Benutzer erblickten Untersuchungsfeldes (9) verlagerbar ist, und wobei auf die Aufforderung (65) hin die Koordinaten des Ortes des Untersuchungsfeldes (9) bestimmt werden, auf den die Markierung (55) zeigt.

12. Verfahren nach Anspruch 10, wobei eine Blickrichtung des Benutzers erfasst wird und auf die Aufforderung hin die Koordinaten des Ortes des Untersuchungsfeldes (9) bestimmt werden, auf den die Blickrichtung des Benutzers weist.

13. Verfahren nach einem der Ansprüche 10 bis 12, wobei das abbildende Gerät ein Mikroskop (11) oder eine Lupenbrille ist.

14. Verfahren nach einem der Ansprüche 1 bis 13, wobei die topologischen Daten oder/und der Koordinatensatz des Ortes mittels einer Kamera oder mehrerer Kameras oder/und optischer Kohärenztomographie oder/und Lasertriangulation oder/und Röntgen-Computertomographie oder/und Kernspinresonanztomographie gewonnen werden.

15. Vorrichtung zum Festlegen wenigstens eines Ortes (59) in einem Untersuchungsfeld (9) bezüglich eines Koordinatensystems (13), umfassend:
eine Zieleinrichtung (35) zur Bestimmung wenigstens eines ersten Ortes (59) in dem Untersuchungsfeld (9) bezüglich des Koordinatensystems (13) und zur Bereitstellung von Koordinaten des ersten Ortes (59) als ein dem ersten Ort (59) zugeordneter erster Koordinatensatz (x, y, z),
eine Topologie-Aufnahmeeinrichtung (37), um eine Aufnahme topologischer Daten (69) in einem räumlich ausgedehnten Bereich um den ersten Ort im Untersuchungsfeld zu gewinnen,
einen Rechner (19), um wenigstens zwei Aufnahmen topologischer Daten miteinander zu vergleichen, aus dem Vergleich eine Verlagerung (Δx, Δy, Δz) des Untersuchungsfeldes bezüglich des Koordinatensystems (13) zu bestimmen und um die Koordinaten des ersten Koordinatensatzes (x, y, z) in Abhängigkeit von der bestimmten Verlagerung (Δx, Δy, Δz) derart abzuändern, daß sie mit den Koordinaten des ersten Orts (59) im Untersuchungsfeld (9) nach dessen Verlagerung im Wesentlichen übereinstimmen .

16. Vorrichtung nach Anspruch 15, wobei die Zieleinrichtung eine Blickrichtungserfassungseinrichtung (35) umfaßt und wobei die Zieleinrichtung zur Bereitstellung der Koordinaten des Ortes vorgesehen ist, auf den der Blick des Benutzers gerichtet ist.

17. Vorrichtung nach Anspruch 15, wobei die Zieleinrichtung eine in ein Blickfeld (47) des Benutzers eingeblendete Anzeige einer Markierung (59) umfaßt und wobei die Zieleinrichtung (35) zur Bereitstellung der Koordinaten des Ortes des Untersuchungsfeldes vorgesehen ist, auf den die Markierung (59) zeigt.

18. Vorrichtung nach Anspruch 15, wobei die Zieleinrichtung einen mit der Hand des Benutzers handhabbaren Zeiger (83) mit einer Zeigerspitze (85) zum Berühren des Ortes des Untersuchungsfeldes (9) und eine Positionserfassungseinrichtung (21, 87) zur Erfassung der Position der Zeigerspitze (85) bezüglich des Koordinatensystems (13) umfaßt.

19. Vorrichtung nach einem der Ansprüche 15 bis 18, wobei die Topologie-Aufnahmeeinrichtung eine Kamera oder/und eine Kohärenztomographievorrichtung (37) oder/und eine Röntgenvorrichtung (81) oder/und eine Magnetresonanz-Tomographievorrichtung (81) umfaßt.

20. Vorrichtung nach einem der Ansprüche 15 bis 19, wobei die Zieleinrichtung (35) zur Bereitstellung von Koordinaten eines zweiten Ortes (55) als ein dem zweiten Ort zugeordneter zweiter Koordinatensatz vorgesehen ist und der Rechner zur Bestimmung eines Abstands (D) zwischen dem ersten und dem zweiten Ort vorgesehen ist.

21. Vorrichtung nach einem der Ansprüche 15 bis 20, ferner umfassend eine Datenerfassungsvorrichtung (37, 81) zur Erfassung von Daten an wenigstens einem in Abhängigkeit von dem ersten Koordinatensatz bestimmten Punkt des Untersuchungsfelds.

22. Vorrichtung nach Anspruch 21, wobei die Zieleinrichtung (35) zur Bereitstellung von Koordinaten wenigstens eines weiteren Ortes als wenigstens ein weiterer Koordinatensatz vorgesehen ist und die Datenerfassungsvorrichtung (37, 81) zur Erfassung von Daten an einer Mehrzahl von Punkten (61) vorgesehen ist, welche in Abhängigkeit von dem ersten und dem wenigstens einen weiteren Koordinatensatz bestimmt sind.

23. Vorrichtung nach Anspruch 22, wobei die Mehrzahl von Punkten entlang einer im Wesentlichen geraden Linie (61) zwischen zwei durch die Zieleinrichtung (35) bestimmten Orten des Untersuchungsfelds angeordnet ist.

24. Vorrichtung nach einem der Ansprüche 15 bis 23, wobei die Datenerfassungsvorrichtung eine Kohärenztomographievorrichtung (37) und/oder eine Röntgenvorrichtung (81) oder/und eine Magnetresonanz-Tomographievorrichtung (81) umfaßt.

25. Vorrichtung nach einem der Ansprüche 15 bis 24, ferner umfassend eine Anzeige (43) für eine Darstellung der durch die Datenerfassungsvorrichtung erfassten Daten in das Blickfeld (47) des Benutzers derart, daß die Darstellung und das Untersuchungsfeld gleichzeitig im Blickfeld (47) des Benutzers sichtbar sind.

26. Vorrichtung nach einem der Ansprüche 15 bis 25, wobei die Anzeige eine Vorrichtung zur Anzeige einer aus einem Untersuchungsfeld (9) gewonnenen Größe (54) umfasst, wobei die Vorrichtung zur Anzeige umfasst:
eine Blickrichtungserfassungseinrichtung (35) zur Erfassung einer auf das Untersuchungsfeld (9) weisenden Blickrichtung eines Benutzers, und
eine Eingabeeinrichtung (63) zur Entgegennahme einer Aufforderung (65, 73) durch den Benutzer,
eine Anzeige (51) zur Darstellung wenigstens einer in Abhängigkeit von dem Koordinatensatz bestimmten Größe (54) in dem Blickfeld (47) des Benutzers derart, daß die Anzeige (54) und das Untersuchungsfeld (9) gleichzeitig im Blickfeld (47) des Benutzers liegen.

27. Vorrichtung nach Anspruch 26, ferner umfassend ein Mikroskop (11) zur Betrachtung des Untersuchungsfeldes (9) durch den Benutzer, wobei die Anzeige (51) zur Darstellung der Größe (54) in einen Strahlengang des Mikroskops (11) eingeblendet ist.

28. Vorrichtung nach Anspruch 27, wobei das Mikroskop (11) relativ zu dem Koordinatensystem (13) verlagerbar ist und eine Positionserfassungseinrichtung (21, 23) zur Erfassung der Position des Mikroskops (11) bezüglich des Koordinatensystems (13) vorgesehen ist.

29. Vorrichtung nach Anspruch 26, wobei die Anzeige eine Halterung zur festen Anbringung an dem Kopf des Benutzers umfaßt und eine Positionserfassungseinrichtung zur Erfassung der Position des Kopfes bezüglich des Koordinatensystems vorgesehen ist.

30. Vorrichtung nach Anspruch 29, wobei die Halterung eine Lupenbrille umfaßt und die Anzeige zur Darstellung der Größe in einen Strahlengang der Lupenbrille eingeblendet ist.

31. Verwendung des Verfahrens nach einem der Ansprüche 1 bis 14 oder/und der Vorrichtung nach einem der Ansprüche 15 bis 30, wobei das Untersuchungsfeld ein medizinisches Operationsfeld ist.

32. Verwendung nach Anspruch 31 zur Bestimmung einer Länge eines Implantats, insbesondere einer Stapesprotese.

## Revendications

1. Procédé pour déterminer au moins un endroit dans un champ d'examen (9) par rapport à un système de coordonnées (13), dans lequel, suite à une sollicitation (65), on détermine les coordonnées d'un premier endroit (59) sous la forme d'un premier jeu de coordonnées (x, y, z) associé au premier endroit (59), et
dans lequel on effectue ensuite successivement un premier et un deuxième relevé de données topologiques (69) dans une zone étendue dans l'espace autour du premier endroit (59) dans le champ d'examen (9),
dans lequel on détermine, à partir d'une comparaison des deux relevés, un déplacement (Δx, Δy, Δz) du premier endroit (59) du champ d'examen (9) par rapport au système de coordonnées (13),
et dans lequel on modifie les coordonnées du premier jeu de coordonnées (x, y, z) associé au premier endroit en fonction du déplacement établi (Δx, Δy, Δz) de façon à ce qu'elles correspondent pour l'essentiel aux coordonnées du premier endroit (59) dans le champ d'examen (9) après le déplacement de celui-ci.

2. Procédé selon la revendication 1, dans lequel les relevés de données topologiques (69) sont effectués de façon périodique et comparés entre eux, comparaisons à partir desquelles on détermine des déplacements (Δx, Δy, Δz) afin d'obtenir les coordonnées actuelles du premier endroit (59) dans le champ d'examen (9).

3. Procédé selon la revendication 1 ou 2, dans lequel on détermine en fonction du premier jeu de coordonnées (x, y, z) au moins une grandeur.

4. Procédé selon la revendication 3, dans lequel s'affiche une représentation (53) de la au moins une grandeur (54) dans le champ visuel (47) d'un utilisateur.

5. Procédé selon l'une des revendications 1 à 4, dans lequel après une autre sollicitation (73), on détermine les coordonnées d'un deuxième endroit (55) sous la forme d'un deuxième jeu de coordonnées associé au deuxième endroit (55) et dans lequel on détermine, en fonction des deux jeux de coordonnées, au moins une grandeur (D).

6. Procédé selon la revendication 5, dans lequel la au moins une grandeur comprend une valeur qui indique essentiellement une distance (D) entre les deux endroits (59, 55).

7. Procédé selon la revendication 5, dans lequel la au moins une grandeur comprend un jeu de données obtenu à partir d'une pluralité de mesures, mesures effectuées le long d'une ligne de jonction (61) entre les deux endroits (59, 55) au niveau du champ d'examen (9).

8. Procédé selon l'une des revendications 1 à 7, dans lequel le relevé de données topologiques comprend des coordonnées d'une pluralité de points de surface (69) du champ d'examen (9).

9. Procédé selon l'une des revendications 1 à 8, dans lequel le relevé de données topologiques comprend des valeurs de mesure de points (69) situés sous la surface du champ d'examen (9).

10. Procédé selon l'une des revendications 1 à 9, dans lequel est représenté au moyen d'un appareil de reproduction (11) une image (49) du champ d'examen (9) destinée à être visualisée par l'utilisateur, ou bien l'utilisateur regarde directement le champ d'examen (9).

11. Procédé selon la revendication 10, dans lequel, pour l'observation par l'utilisateur, il est en outre représenté un repère (55) dont la position peut être déplacée par rapport au champ d'examen (9) observé par l'utilisateur, et dans lequel, sur demande (65), il est indiqué les coordonnées de l'endroit du champ d'examen (9) indiqué par le repère (55).

12. Procédé selon la revendication 10, dans lequel on enregistre la direction de visée de l'utilisateur et, sur demande, on détermine les coordonnées de l'endroit du champ d'examen (9) sur lequel se porte le regard de l'utilisateur.

13. Procédé selon l'une des revendications 10 à 12, dans lequel l'appareil de reproduction est un microscope (11) ou une lunette-loupe.

14. Procédé selon l'une des revendications 1 à 13, dans lequel les données topologiques et/ou le jeu de coordonnées de l'endroit sont obtenus à l'aide d'une caméra ou de plusieurs caméras et/ou par tomographie à cohérence optique et/ou par triangulation laser et/ou par tomodensitométrie et/ou par tomographie à résonance magnétique nucléaire.

15. Dispositif pour définir au moins un endroit (59) dans un champ d'examen (9) par rapport à un système de coordonnées (13), comprenant :
un système de visée (35) pour déterminer au moins un endroit (59) dans le champ d'examen (9) par rapport au système de coordonnées (13) et pour fournir les coordonnées du premier endroit (59) sous la forme d'un premier jeu de coordonnées (x, y, z) associé au premier endroit (59),
un dispositif de relevé topologique (37) pour obtenir un relevé de données topologiques (69) dans une zone étendue dans l'espace autour du premier endroit dans le champ d'examen,
un ordinateur (19) pour pouvoir comparer au moins deux relevés de données topologiques entre eux, comparaison à partir de laquelle on détermine un déplacement (Δx, Δy, Δz) du champ d'examen par rapport au système de coordonnées (13) et pouvoir ainsi modifier les coordonnées du premier jeu de coordonnées (x, y, z) en fonction du déplacement établi (Δx, Δy, Δz) de façon à ce qu'elles correspondent pour l'essentiel aux coordonnées du premier endroit (59) dans le champ d'examen (9) après le déplacement de celui-ci.

16. Dispositif selon la revendication 15, dans lequel le dispositif de visée comprend un dispositif de détection de la direction de visée (35) et dans lequel le dispositif de visée est prévu pour fournir les coordonnées de l'endroit sur lequel se porte le regard de l'utilisateur.

17. Dispositif selon la revendication 15, dans lequel le dispositif de visée comprend l'affichage d'un repère (59) s'affichant dans le champ visuel (47) de l'utilisateur, et dans lequel le dispositif de visée (35) est prévu pour fournir les coordonnées de l'endroit du champ d'examen montré par le repère (59).

18. Dispositif selon la revendication 15, dans lequel le dispositif de visée comprend un pointeur (83) pouvant être manipulé à l'aide de la main de l'utilisateur avec une pointe d'aiguille (85) destinée à venir toucher l'endroit du champ d'examen (9) et un dispositif de détection de la position (21, 87) pour déterminer la position de la pointe d'aiguille (85) par rapport au système de coordonnées (13).

19. Dispositif selon l'une des revendications 15 à 18, dans lequel le dispositif de relevé topologique comprend une caméra et/ou un dispositif de tomographie à cohérence (37) et/ou un dispositif à rayons X (81) et/ou un dispositif de tomographie par résonance magnétique (81).

20. Dispositif selon l'une des revendications 15 à 19, dans lequel le dispositif de visée (35) est prévu pour fournir les coordonnées d'un deuxième endroit (55) sous la forme d'un deuxième jeu de coordonnées associé au deuxième endroit et dans lequel l'ordinateur est prévu pour déterminer une distance (D) entre le premier et le deuxième endroit.

21. Dispositif selon l'une des revendications 15 à 20, comprenant en outre un dispositif d'enregistrement de données (37, 81) pour enregistrer des données au niveau d'au moins un point du champ d'examen défini en fonction du premier jeu de coordonnées.

22. Dispositif selon la revendication 21, dans lequel le dispositif de visée (35) est prévu pour fournir les coordonnées d'au moins un autre endroit sous la forme d'au moins un autre jeu de coordonnées et dans lequel le dispositif d'enregistrement de données (37, 81) est prévu pour enregistrer des données au niveau d'une pluralité de points (61), lesquels sont déterminés en fonction du premier jeu de coordonnées et du au moins un autre jeu de coordonnées.

23. Dispositif selon la revendication 22, dans lequel la pluralité de points est agencée le long d'une ligne essentiellement droite (61) entre deux endroits du champ d'examen définis par le dispositif de visée (35).

24. Dispositif selon l'une des revendications 15 à 23, dans lequel le dispositif d'enregistrement de données comprend un dispositif de tomographie à cohérence (37) et/ou un dispositif à rayons X (81) et/ou un dispositif de tomographie par résonance magnétique (81).

25. Dispositif selon l'une des revendications 15 à 24, comprenant en outre un affichage (43) pour la représentation des données collectées par le dispositif d'enregistrement de données dans le champ visuel (47) de l'utilisateur de telle sorte que la représentation et le champ d'examen sont visibles simultanément dans le champ visuel (47) de l'utilisateur.

26. Dispositif selon l'une des revendications 15 à 25, dans lequel l'affichage comprend un dispositif pour afficher une dimension (54) obtenue à partir d'un champ d'examen (9), le dispositif d'affichage comprenant :
un dispositif d'enregistrement de la direction de visée (35) pour enregistrer la direction de visée d'un utilisateur orientée vers le champ d'examen (9), et
un dispositif d'entrée (63) pour la réception d'une sollicitation (65, 73) par l'utilisateur,
un affichage (51) pour la représentation d'au moins une grandeur (54) définie en fonction du jeu de coordonnées dans le champ visuel (47) de l'utilisateur de façon à ce que l'affichage (54) et le champ d'examen (9) soient représentés simultanément dans le champ visuel (47) de l'utilisateur.

27. Dispositif selon la revendication 26, comprenant en outre un microscope (11) pour l'examination du champ d'examen (9) par l'utilisateur, l'affichage (51) pour représenter la grandeur (54) apparaissant dans une trajectoire de faisceau du microscope (11).

28. Dispositif selon la revendication 27, dans lequel le microscope (11) peut être déplacé par rapport au système de coordonnées (13) et dans lequel il est prévu un dispositif d'enregistrement de la position (21, 23) pour enregistrer la position du microscope (11) par rapport au système de coordonnées (13).

29. Dispositif selon la revendication 26, dans lequel l'affichage comprend un support pour pouvoir être fixement posé sur la tête de l'utilisateur et dans lequel il est prévu un dispositif d'enregistrement de la position pour enregistrer la position de la tête par rapport au système de coordonnées.

30. Dispositif selon la revendication 29, dans lequel le support comprend une lunette-loupe et l'affichage pour représenter la grandeur apparaît dans un faisceau de la lunette-loupe.

31. Utilisation du procédé selon l'une des revendications 1 à 14 et/ou du dispositif selon l'une des revendications 15 à 30, dans laquelle le champ d'examen est un champ d'opération médical.

32. Utilisation selon la revendication 31 pour déterminer une longueur d'un implant, en particulier d'une prothèse d'étrier.

## Claims

1. Method for fixing at least one location in an examination field (9) in respect of a coordinate system (13), wherein, in response to a request (65), coordinates of a first location (59) are determined as a first coordinate set (x, y, z) allocated to the first location (59), and, subsequently,
a first recording and a second recording of topological data (69) are successively obtained in a spatially extended area around the first location (59) in the examination field (9),
on the basis of a comparison of the two recordings, a displacement (Δx, Δy, Δz) of the first location (59) of the examination field (9) in respect of the coordinate system (13) is determined, and
the coordinates of the first coordinate set (x, y, z) allocated to the first location are changed in depencence on the determined displacement (Δx, Δy, Δz) such that they substantially correspond to the coordinates of the first location (59) in the examination field (9) after the displacement thereof.

2. Method according to claim 1, wherein the recordings of topological data (69) are periodically obtained and compared with one another and displacements (Δx, Δy, Δz) are determined therefrom to obtain current coordinates of the first location (59) in the examination field (9).

3. Method according to claim 1 or 2, wherein at least one value is determined in depencence on the first coordinate set (x, y, z).

4. Method according to claim 3, wherein a representation (53) of the at least one value (54) is faded into a field of view (47) of a user.

5. Method according to any one of claims 1 to 4, wherein, in response to a further request (73), coordinates of a second location (55) are determined as a second coordinate set allocated to the second location (55) and at least one value (D) is determined in depencence on the two coordinate sets.

6. Method according to claim 5, wherein the at least one value includes a value which substantially indicates a distance (D) between the two locations (59, 55).

7. Method according to claim 5, wherein the at least one value includes a data set obtained from a plurality of measurements taken along a connecting line (61) between the two locations (59, 55) of the examination field (9).

8. Method according to any one of claims 1 to 7, wherein the recording of the topological data comprises coordinates of a plurality of surface points (69) of the examination field (9).

9. Method according to any one of claims 1 to 8, wherein the recording of the topological data comprises measured values of points (69) below the surface of the examination field (9).

10. Method according to any one of claims 1 to 9, wherein an image (49) of the examination field (9) to be viewed by the user is displayed by means of an imaging device (11), or the user looks directly at the examination field (9).

11. Method according to claim 10, wherein, furthermore, a mark (55) to be viewed by the user is displayed, the position of said mark being displaceable in respect of the examination field (9) viewed by the user and wherein, in response to the request (65), the coordinates of the location of the examination field (9) to which the mark (55) points are determined.

12. Method according to claim 10, wherein a line of sight of the user is detected and, in response to the request, the coordinates of the location of the examination field (9) is determined to which the line of sight of the user is directed.

13. Method according to any one of claims 10 to 12, wherein the imaging device is a microscope (11) or a binocular loupe.

14. Method according to any one of claims 1 to 13, wherein the topological data or/and the coordinate set of the location are obtained by means of a camera or several cameras or/and optical coherence tomography or/and laser triangulation or/and X-ray computer tomography or/and nuclear magnetic resonance tomography.

15. Apparatus for fixing at least one location (59) in an examination field (9) in respect of a coordinate system (13), comprising:
a targeting device (35) for determining at least a first location (59) in the examination field (9) in respect of the coordinate system (13) and for providing coordinates of the first location (59) as a first coordinate set (x, y, z) allocated to the first location (59),
a topology recording device (37) for obtaining a recording of topological data (69) in a spatially extended area around the first location in the examination field,
a computer (19) for comparing at least two recordings of topological data, for determining, on the basis of the comparison, a displacement (Δx, Δy, Δz) of the examination field in respect of the coordinate system (13) and for changing the coordinates of the first coordinate set (x, y, z) in depencence on the determined displacement (Δx, Δy, Δz) such that they substantially correspond to the coordinates of the first location (59) in the examination field (9) after the displacement thereof.

16. Apparatus according to claim 15, wherein the targeting device comprises a line-of-sight detecting device (35) and the targeting device is provided for providing the coordinates of the location to which the eyes of the user are directed.

17. Apparatus according to claim 15, wherein the targeting device comprises a display of a marking (59) which is faded into a field of view (47) of the user, and wherein the targeting device (35) is provided for providing the coordinates of the location of the examination field at which the marking (59) points.

18. Apparatus according to claim 15, wherein the targeting device comprises a pointer (83) which may be manipulatable by the user's hand and includes a pointer tip (85) for contacting the location of the examination field (9), and a position detecting device (21, 87) for detecting the position of the pointer tip (85) in respect of the coordinate system (13).

19. Apparatus according to any one of claims 15 to 18, wherein the topology recording device comprises a camera or/and a coherence tomography apparatus (37) or/and an X-ray apparatus (81) or/and a magnetic resonance tomography apparatus (81).

20. Apparatus according to any one of claims 15 to 19, wherein the targeting device (35) is provided for providing coordinates of a second location (55) as a second coordinate set allocated to the second location and the computer is provided for determining a distance (D) between the first and the second location.

21. Apparatus according to any one of claims 15 to 20, further comprising a data recording apparatus (37, 81) for recording data at at least one point of the examination field determined in depencence on the first coordinate set.

22. Apparatus according to claim 21, wherein the targeting device (35) is provided for providing coordinates of at least one further location as at least one further coordinate set and the data recording apparatus (37, 81) is provided for recording data at a plurality of points (61) which are determined in depencence on the first and the at least one further coordinate set.

23. Apparatus according to claim 22, wherein the plurality of points are positioned along a substantially straight line (61) between two locations of the examination field determined by the targeting device (35).

24. Apparatus according to any one of claims 15 to 23, wherein the data recording apparatus is a coherence tomography apparatus (37) and/or an X-ray apparatus (81) or/and a magnetic resonance tomography apparatus (81).

25. Apparatus according to any one of claims 15 to 24, further comprising a display (43) for displaying the data recorded by the data recording apparatus in the field of view (47) of the user such that the display and the examination field are simultaneously visible in the field of view (47) of the user.

26. Apparatus according to any one of claims 15 to 25, wherein the display comprises an apparatus for displaying a value (54) obtained from an examination field (9), comprising:
a line-of-sight detecting device (35) for detecting a line of sight of a user which is directed to the examination field (9),
an input device (63) for receiving a request (65, 73) made by the user,
a display (51) for displaying at least one value (54) determined in depencence on the coordinate set in the field of view (47) of the user such that the display (54) and the examination field (9) are simultaneously in the field of view (47) of the user.

27. Apparatus according to claim 26, further comprising a microscope (11) for viewing the examination field (9) by the user, wherein the display (51) for displaying the value (54) is faded into a beam path of the microscope (11).

28. Apparatus according to claim 27, wherein the microscope (11) is displaceable relative to the coordinate system (13) and a position detecting device (21, 23) is provided for detecting the position of the microscope (11) in respect of the coordinate system (13).

29. Apparatus according to claim 26, wherein the display comprises a support to be fixedly secured at the head of the user and a position detecting device is provided for detecting the position of the head in respect of the coordinate system.

30. Apparatus according to claim 29, wherein the support comprises a binocular loupe and the display for displaying the value is faded into a beam path of the binocular loupe.

31. Use of the method according to any one of claims 1 to 14 or/and of the apparatus according to any one of claims 15 to 30, wherein the examination field is a surgical operation field.

32. Use according to claim 31 for determining a length of an implant, in particular of a stapes prosthesis.
